# EUROPÄISCHE PATENTSCHRIFT

(11) **EP 1 919 559 B1**
(45) Veröffentlichungstag und Bekanntmachung des Hinweises auf die Patenterteilung: **27.10.2010**
(21) Anmeldenummer: 06762681.2
(22) Anmeldetag: 19.07.2006
(51) Int. Cl.: A61N 5/06

(54) **STRAHLEREINRICHTUNG**
RADIATOR DEVICE
DISPOSITIF DE RAYONNEMENT

(30) Priorität: 09.08.2005 DE 102005037453
(43) Veröffentlichungstag der Anmeldung: 14.05.2008
(73) Patentinhaber: uwe GmbH, 73525 Schwäbisch Gmünd (DE)
(72) Erfinder: KUHNER, Konrad, 73510 Schwäbisch-Gmünd (DE)
(74) Vertreter: Gleiss, Alf-Olav
(86) Internationale Anmeldenummer: PCT/EP2006/007079
(87) Internationale Veröffentlichungsnummer: WO 2007/017062

(56) Entgegenhaltungen:
- DE-U1- 29 517 716
- FR-A- 2 585 957
- NL-A- 9 000 302
- US-B1- 6 494 899

## Beschreibung

Die Erfindung betrifft eine Strahlereinrichtung einer Bräunungs- und/oder Wärmestrahlervorrichtung gemäß Oberbegriff des Anspruchs 1. Eine solche Strahlereinrichtung wird in der Patentanmeldung FR 2 585 957 A1 beschrieben.

Strahlereinrichtungen der hier angesprochenen Art sind bekannt. Sie werden beispielsweise in Bräunungsvorrichtungen unterschiedlichster Art oder aber in Wärmequellen verwendet. Es hat sich als nachteilig herausgestellt, dass die mindestens eine Strahlungsquelle einen Strahlungskegel aufweist, der eine mehr oder weniger große Fläche eines Benutzers der Strahlungseinrichtung bestrahlt. Dies schränkt die Nutzungsmöglichkeiten der Strahlungseinrichtung ein. Ist nämlich die Strahlungsquelle als Bräunungsstrahler ausgebildet, so ist zu vermeiden, dass der mit der Strahlung beaufschlagte Körperbereich keine zu hohe Strahlungsintensität erhält. Andererseits zeigt es sich, dass in vielen Fällen relativ scharfrandige Bräunungsbereiche entstehen, die hohen ästhetischen Anforderungen der Benutzer solcher Strahlungseinrichtungen nicht genügen können. Wenn die Strahlungsquelle einer derartigen Strahlungseinrichtung als Wärmestrahler ausgelegt ist, ist es für den Benutzer häufig unangenehm, wenn ein einzelner Bereich des Körpers zu sehr erwärmt wird.

Aufgabe der Erfindung ist es daher, eine Strahlungseinrichtung zu schaffen, die diese Nachteile vermeidet.

Zur Lösung dieser Aufgabe wird eine Strahlungsquelle vorgeschlagen, die die in Anspruch 1 genannten Merkmale aufweist. Sie umfasst eine Halterung und mindestens eine dieser zugeordnete Strahlungsquelle. Die Strahleinrichtung zeichnet sich durch einen Antrieb auf, der eine Relativbewegung zwischen der Halterung und der mindestens einen Strahlungsquelle bewirkt. Der von der mindestens einen Strahlungsquelle erzeugte Strahlungskegel trifft also nicht auf einen von der Strahlungsgeometrie der Strahlungsquelle bestimmten Punkt des Körpers des Benutzers. Vielmehr wird der Strahlungskegel mittels des Antriebs durch die Relativbewegung zwischen Strahlungsquelle und Halterung bewegt, sodass ein größerer Bereich des Körpers des Benutzers von dem Strahlungskegel beaufschlagt wird. Die Strahlungsenergie einer Strahlungsquelle verteilt sich damit auf einen größeren Körperbereich, sodass einerseits bei Verwendung der Strahlungseinrichtung in Zusammenhang mit einer Bräunungsquelle keine scharfrandigen Bräunungsbereiche entstehen, andererseits die Gefahr einer Schädigung der Haut eines Benutzers durch die Bewegung des Strahlungskegels reduziert wird. Bei einer derartig ausgebildeten Strahlungseinrichtung einer Wärmestrahlervorrichtung wird sichergestellt, dass sich nicht nur eine quasi punktförmige Erwärmung der Haut eines Benutzers ergibt, sondern ein größerer Bereich mit Wärme beaufschlagt wird, was für den Benutzer angenehmer ist.

Bei einem bevorzugten Ausführungsbeispiel der Strahlungseinrichtung ist vorgesehen, dass der Antrieb als Schwenk-, Kreis-, Kreisel- oder Taumelantrieb auslegbar ist. Es zeigt sich also, dass die Strahlereinrichtung flexibel an verschiedene Anwendungsfälle anpassbar ist.

Ein weiteres Ausführungsbeispiel zeichnet sich dadurch aus, dass der Antrieb einen Motor umfasst. Dabei ist es möglich, die Antriebswelle des Motors im Endbereich gegenüber der Drehachse etwas zu verschwenken und unmittelbar mit der Strahlungsquelle zu verbinden. Auf einfache Weise kann so beispielsweise eine Kreisbewegung des Strahlungskegels realisiert werden, sodass sich die oben genannten Vorteile einstellen. Der Motor kann aber auch Teil eines aufwendigeren Antriebs sein, der auch höheren Anforderungen gerecht wird und andere Bewegungsabläufe der Strahlungsquelle ermöglicht.

Weitere Ausgestaltungen ergeben sich aus den übrigen Unteransprüchen.

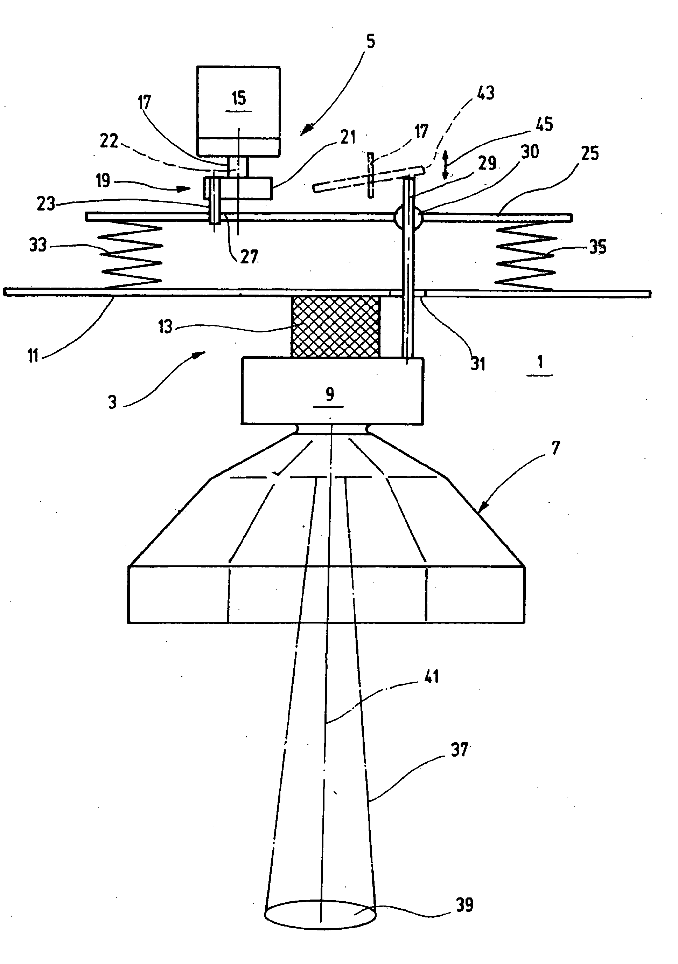

Die Erfindung wird im Folgenden anhand einer Zeichnung näher erläutert. Dabei zeigt die einzige Figur eine Prinzipskizze einer Strahlereinrichtung in Seitenansicht.

Aus der Figur ist eine Strahlereinrichtung 1 ersichtlich, die eine Halterung 3 sowie einen Antrieb 5 umfasst, außerdem hier eine Strahlungsquelle 7.

Die Strahlungseinrichtung 1 ist Teil einer Bräunungs- und/oder Wärmestrahlervorrichtung und ist entsprechend ausgelegt: sie kann als reiner Ultraviolett-Strahler (UV-Strahler) oder als reiner InfrarotStrahler (IR-Strahler) ausgelegt sein. Denkbar ist es aber auch, einen UV-Strahler so auszulegen, dass er zusätzlich auch Wärmestrahlung abgibt.

Bei dem hier dargestellten Ausführungsbeispiel weist die Strahlungsquelle 7 einen Sockel 9 auf. Dieser ist mit der Halterung 3 beweglich verbunden. Dazu ist hier vorgesehen, dass die Halterung 3 mindestens ein Trägerelement 11 aufweist, an dem ein elastisches Verbindungselement 13 angebracht ist. Dieses ist mit dem Sockel 9 der Strahlungsquelle 1 verbunden.

Aufgrund dieser Anbringung ist gewährleistet, dass die Strahlungsquelle 7 gegenüber dem Trägerelement 11 beweglich ist. Denkbar ist es, als elastisches Verbindungselement 13 ein elastisches Gummi- oder Kunststoffelement einzusetzen. Möglich ist es auch, als elastisches Verbindungselement 13 einen Blechstreifen oder ein Federelement, insbesondere eine Schraubenfeder, zu verwenden, die einerseits am Sockel 9 und andererseits am Trägerelement 11 angebracht ist. Entscheidend ist, dass die Strahlungsquelle 7 gegenüber dem Trägerelement 11 beweglich ist und vorzugsweise eine Schwenk-, Kreisel-, Kreis- oder Taumelbewegung durchführen kann.

Die Relativbewegung zwischen dem Trägerelement 11 und der Strahlungsquelle 7 wird durch den Antrieb 5 bewirkt. Es wird deutlich, dass zur Realisierung dieser Bewegung die Strahlungsquelle 7 auch unmittelbar mit dem elastischen Verbindungselement 13 gekoppelt werden kann, dass also der Sockel 9 verzichtbar ist.

Bei dem hier dargestellten Ausführungsbeispiel weist der Antrieb 5 einen Motor 15 auf, der beispielsweise als Elektromotor ausgelegt und mit einer entsprechenden Energiequelle verbunden ist. Insbesondere wird dabei vorgesehen, dass der Motor 15 so ansteuerbar ist, dass nicht nur eine Ein- oder Ausschaltung möglich ist, sondern auch eine Steuerung oder Regelung der Drehzahl und/oder Drehrichtung des Motors 5.

An der Antriebswelle 17 des Motors 15 ist ein Exzenter 19 angekoppelt, der hier eine mit der Antriebswelle 17 drehfest verbundene Exzenterscheibe 21 umfasst, in die in einem Abstand zur Drehachse 22 der Antriebswelle 17 ein Kopplungsstift 23 eingesetzt ist. Es wird deutlich, dass anstelle einer im Wesentlichen runden oder ovalen Exzenterscheibe 21 auch ein mit der Antriebswelle 17 gekoppelter Arm vorgesehen werden kann, in den in einem Abstand zur Drehachse 22 der Antriebswelle 17 der Kopplungsstift 23 eingesetzt ist. Es zeigt sich im Übrigen, dass der Exzenter 19 auch dadurch realisierbar ist, dass die Antriebswelle 17 so umgebogen wird, dass ein in einem Winkel zur Drehachse 22 verlaufender Arm ausgebildet wird, dessen Ende -hier nach unten- umgebogen ist, um den nicht mit der Drehachse 22 der Antriebswelle 17 zusammenfallenden Kopplungsstift 23 zu realisieren.

Bei dem in der Figur wiedergegebenen Ausführungsbeispiel greift der Kopplungsstift 23 in eine Mitnehmerplatte 25 ein. Dreht sich die Antriebswelle 17 des von einer geeigneten Halterung drehfest gehaltenen Motors 15, so bewegt sich der Kopplungsstift 23 auf einer konzentrisch zur Antriebswelle 17 liegenden Kreisbahn. Da der Kopplungsstift 23 mit der Mitnehmerplatte 25 drehbar verbunden ist, beispielsweise durch ein in der Mitnehmerplatte 25 vorgesehenes Loch 27 geführt ist, bewegt sich zumindest das Loch 27 der Mitnehmerplatte 25 ebenfalls auf einer Kreisbahn, die konzentrisch zur Antriebswelle 17 liegt.

Der Antrieb 5 umfasst außerdem einen Mitnehmer 29, der mit der Mitnehmerplatte 25 beweglich gekoppelt ist und an der Strahlungsquelle 7, hier an deren Sockel 9, angreift. Der mit der Strahlungsquelle 7 gekoppelte Mitnehmer 29 führt innerhalb der Mitnehmerplatte 25 eine Kippbewegung durch. Die Mitnehmerplatte 25 übt hier im Wesentlichen senkrecht zur Längserstreckung des Mitnehmers 29 wirkende Kräfte auf den Mitnehmer 29 aus. Um Reibungsverluste zu vermeiden und um den Verschleiß zu reduzieren, ist der Mitnehmer 29 mit der Mitnehmerplatte 25 über eine Kalotte 30 gekoppelt. Eine Bewegung der Mitnehmerplatte 25 wird also von dem Mitnehmer 29 auf den Strahler 7 übertragen. Dazu greift der Mitnehmer 29 durch eine Öffnung 31 im Trägerelement 11. Bei dem hier dargestellten Ausführungsbeispiel befinden sich die Mitnehmerplatte 25 auf einer ersten Seite des Trägerelements 11 und der Strahler 7 auf dessen gegenüberliegenden Seite. Die Mitnehmerplatte 25 ist auf geeignete Weise an einer Halterung beweglich angebracht. Hier ist vorgesehen, dass die Mitnehmerplatte 25 über elastische Elemente 33 und 35 mit dem Trägerelement 11 gekoppelt sind. Denkbar ist es aber auch, die Mitnehmerplatte 25 mit einer oberhalb derselben liegenden Halterung zu koppeln, die beispielsweise auch dazu dienen kann, den Motor 15 drehfest zu fixieren. Die elastischen Elemente 33 und 35 können aus Gummi, Kunststoff bestehen, oder aber, wie hier angedeutet, als Federelemente realisiert sein.

Nach allem zeigt sich, dass der Antrieb 5 hier so ausgelegt ist, dass bei einer Aktivierung des Motors 15 dessen Antriebswelle 17 in Rotation versetzt wird, die über den Exzenter 19 auf die Mitnehmerplatte 25 übertragen wird. Deren Bewegung wird über den Mitnehmer 25 auf die Strahlungsquelle 7 übertragen, hier über deren Sockel 9. Die Strahlungsquelle 7 führt hier eine Taumelbewegung durch, sodass der Strahlungskegel 37 der Strahlungsquelle 7 nicht nur einen festen Strahlungsfleck 39 beispielsweise auf der Körperoberfläche eines Benutzers liefert. Vielmehr wird der Strahlungskegel 37 entsprechend der Taumelbewegung der Strahlungsquelle 7 bewegt, damit auch der Strahlungsfleck 39, der damit einen Bereich der Körperoberfläche eines Benutzers überstreicht.

Aus der Figur ist ersichtlich, dass der Mitnehmer 29 in einem Abstand zum elastischen Verbindungselement 13 am Sockel angebracht ist. Es ist also möglich, den Sockel dadurch zu verkippen, dass auf den Mitnehmer 29 im Wesentlichen senkrecht von oben Kräfte ausgeübt werden. Diese können durch eine Exzenterscheibe erzeugt werden, deren Drehachse im Wesentlichen senkrecht zur Längserstreckung des Mitnehmers verläuft. Der Motor 15 wäre hier, sofern kein Getriebe eingesetzt wird, um 90° im oder gegen den Uhrzeigersinn verschwenkt. Eine weitere Möglichkeit, senkrecht von oben Kräfte auf den Mitnehmer auszuüben wäre, eine unter einem Winkel zur Drehachse 22 der Antriebswelle 17 angeordnete Scheibe an der Antriebswelle 17 anzubringen. Diese führt bei einer Drehbewegung der Antriebswelle 17 des Motors 15 quasi eine Wobbelbewegung durch. Diese Scheibe kann so angeordnet werden, dass sie von oben auf den Mitnehmer 29 wirkt und diesen mehr oder weniger weit nach unten drückt, so dass die Strahlungsquelle 7 eine Relativbewegung gegenüber dem Trägerelement 11 durchführt. Hier wird im Wesentlichen eine Schwenkbewegung der Strahlungsquelle 7 realisiert.

In der Figur ist eine derartige unter einem Winkel zur Antriebswelle 17 eines hier nicht dargestellten Antriebs angeordnete Scheibe 43 gestrichelt angedeutet. Die Auf- und Abbewegung des Mitnehmers 29, die durch eine derartige Scheibe 43 erzeugt wird, wird durch einen Doppelpfeil 45 angedeutet.

Aus den Erläuterungen zu der Strahlereinrichtung 1 wird deutlich, dass die Strahlungsquelle 7 beweglich mit dem Trägerelement 1 gekoppelt ist. Der Antrieb 5 kann so ausgelegt werden, dass die Strahlungsquelle 7 lediglich eine Schwenkbewegung ausführt, sodass der Strahlungsfleck 39 auf der Oberfläche des Körpers eines Benutzers quasi einen Streifen überstreicht. Er kann aber, wie hier erläutert, auch so ausgebildet sein, dass die Strahlungsquelle 7, damit auch deren Strahlungskegel 37 eine Taumelbewegung durchführt, sodass der Strahlungsfleck 39 der Strahlungsquelle 7 einen Bereich auf der Körperoberfläche eines Benutzers überstreicht, der größer ist als der Strahlungsfleck 39 selbst.

Es wird im Übrigen deutlich, dass die Strahlungsquelle 7 auch unmittelbar mit der Antriebswelle 17 eines Motors 15 gekoppelt werden kann, wobei das Ende der Antriebswelle 17 gegenüber deren Mittel- und Drehachse unter einem Winkel verbogen ist. Damit wird die Strahlungsquelle 7 bei Aktivierung des Motors 15 kreiselförmig so bewegt, dass die Mittelachse 41 des Strahlungskegels 37 auf einem Kegelmantel liegt und der Strahlungsfleck 39 auf einer Kreisbahn bewegt wird. Eine Bewegung des Strahlungsflecks 39 kann auch dadurch bewirkt werden, dass die Antriebswelle 17 des Motors 15 geknickt ist und zwei im Wesentlichen parallel oder unter einem Winkel zueinander verlaufende Bereiche aufweist. Ein erster liegt vorzugsweise konzentrisch zur Drehachse 22 der Antriebswelle, ein zweiter seitlich versetzt dazu. Bei dieser Ausführungsform des Motors 15 bewegt sich der Strahlungsfleck 39 auf einer Kreisbahn.

In allen Fällen wird verhindert, dass die Strahlungsquelle 7 auf der Körperoberfläche eines Benutzers einen feststehenden Strahlungsfleck 39 realisiert, der einerseits zu einem möglicherweise relativ scharfrandig umschriebenen gebräunten Fleck führen kann, sofern die Strahlereinrichtung 1 Teil einer Bräunungsvorrichtung ist, andererseits aber auch zu einer lokalen Überwärmung führt, falls die Strahlereinrichtung 1 Teil einer Wärmestrahlervorrichtung ist.

Den Erläuterungen zum Antrieb 5 ist zu entnehmen, dass die Mitnehmerplatte 25 auch quasi stabförmig ausgebildet werden kann, wobei dieser Stab einerseits mit mindestens einem elastischen Element 33 oder 35, dem Kopplungsstift 23 des Exzenters 19 und dem Mitnehmer 29 verbunden ist. Die Mitnehmerplatte 25 kann aber auch so groß ausgebildet werden, dass mehrere Mitnehmer 29 an dieser eingreifen, wobei die mehreren Mitnehmer 29 mehreren Strahlungsquellen 7 zugeordnet sind. Es ist also möglich, mit einem Motor 15 mehrere Strahlungsquellen 7 so anzuregen, dass diese eine Schwenk-, Kreisel- beziehungsweise Kreis- oder Taumelbewegung durchführen.

Die Strahlereinrichtung 1 kann auch mehrere an einem Trägerelement 11 befestigte Strahlungsquellen 7 aufweisen, die von einem einzigen Antrieb 5 oder von mehreren getrennten Antrieben bewegt werden. Darüber hinaus ist es möglich, mehrere getrennte Trägerelemente vorzusehen, die jeweils eine oder mehrere Strahlungsquellen 7 aufweisen. Auch hier ist es denkbar, alle Strahlungsquellen 7 mit einem einzigen Antrieb 5 zu bewegen oder jeden Trägerelement 11 mindestens einen Antrieb 5 zuzuordnen.

Wird eine Strahlereinrichtung 1 mit mehreren Strahlern realisiert, so ist es möglich, diese alle einzeln anzusteuern und für jede Strahlungsquelle 7 einen Motor 15 vorzusehen. Ein besonders einfacher Aufbau des Antriebs 5 ist dadurch erzielbar, dass der Mitnehmer 29 an Stelle des Kopplungsstifts 23 mit dem Exzenter 19 gekoppelt wird, beispielsweise mit der Exzenterscheibe 21. Bei einer Drehung der Exzenterscheibe 21 läuft also das Ende des Mitnehmers 29 auf einer Kreisbahn um die Drehachse 22 der Antriebswelle 17 des Motors 15 um. Die Bewegung des Mitnehmers 29 wird auf den Sockel 9 oder unmittelbar auf die Strahlungsquelle 7 übertragen, so dass diese eine Relativbewegung gegenüber dem Trägerelement 11 ausführt. Dieses Ausführungsbeispiel zeichnet sich dadurch aus, dass auf die Mitnehmerplatte 25 und das mindestens eine elastische Element 33, 35 verzichtet werden kann. Dadurch ergibt sich ein relativ kompakter Aufbau eines Antriebs 5 für jede Strahlungsquelle 7. Besonders vorteilhaft ist es dabei, dass die Motoren 15 unabhängig voneinander ansteuerbar sind, also unabhängig ein- und abschaltbar und mit verschiedenen Drehzahlen und Drehrichtungen antreibbar sind.

Insgesamt ist es möglich, eine Strahlereinrichtung 1 auf vielfache Weise zu realisieren: Es kann eine einzige Strahlungsquelle 7 mit einem Antrieb 5 gekoppelt werden, wie dies die Figur zeigt. Denkbar ist es auch, mehrere Strahlungsquellen mit einem Antrieb zu kombinieren oder aber einer Vielzahl von Strahlungsquellen einen oder mehrere Antriebe zuzuordnen, wobei jeder Antrieb auch mehr als eine Strahlungsquelle bewegen kann. Dabei ist es auch möglich, dass eine Anzahl von Strahlungsquellen einer Strahlungseinrichtung unterschiedliche Bewegungen durchführen, dass also Schwenk-, Kreisel-, Kreis- und Taumelbewegungen innerhalb einer Strahlungseinrichtung 1 realisierbar sind.

Die Strahlereinrichtung 1 kann in einem separaten Gesichts-Bräunungsstrahler in einem Solarium verwendet werden, wobei dann alle Strahler beweglich realisierbar sind, oder aber auch nur einzelne, beispielsweise auch hier einsetzbare Gesichts-Bräunungsstrahler. Darüber hinaus kann die Strahlereinrichtung 1 in Zusammenhang mit so genannten Sonnenwiesen verwendet werden. Schließlich können Strahlereinrichtungen der hier beschriebenen Art auch in Fitness-Centern eingesetzt werden.

Den Erläuterungen zu der Figur ist zu entnehmen, dass die Strahlereinrichtung 1 auch sehr einfach und kompakt aufgebaut sein kann. Damit ist es möglich, bestehende Bräunungs- und/oder Wärmestrahlervorrichtung mit einer Strahlungseinrichtung der hier beschriebenen Art nachzurüsten.

## Patentansprüche

1. Strahlereinrichtung einer Bräunungs- und/oder Wärmestrahlervorrichtung mit einer Halterung (3) und mit mindestens einer der Halterung (3) zugeordneten Strahlungsquelle (7), und mit einem Antrieb (5), der eine Relativbewegung zwischen der mindestens einen Strahlungsquelle (7) und der Halterung (3) bewirkt, **dadurch gekennzeichnet, dass** der Antrieb (5) als Schwenk-, Kreis-, Kreisel- oder Taumelantrieb auslegbar ist und einen Exzenter (19) umfasst, der zumindest über einen Mitnehmer (29) an der Strahlungsquelle (7) angreift.

2. Strahlereinrichtung nach Anspruch 1, **dadurch gekennzeichnet, dass** der Antrieb (5) einen Motor (15) umfasst.

3. Strahlereinrichtung nach Anspruch 2, **dadurch gekennzeichnet, dass** der Exzenter (19) durch eine speziell ausgebildete Antriebswelle (17) des Motors (25) realisierbar ist.

4. Strahlereinrichtung nach Anspruch 3, **dadurch gekennzeichnet, dass** der Exzenter (19) eine Mitnehmerplatte (25) antreibt.

5. Strahlereinrichtung nach einem der vorhergehenden Ansprüche, **dadurch gekennzeichnet, dass** die Halterung (3) mindestens ein Trägerelement (11) und ein elastisches Verbindungselement (13) aufweist, das am Trägerelement (11) und an der mindestens einen Strahlungsquelle (7) angebracht ist.

6. Strahlereinrichtung nach einem der vorhergehenden Ansprüche, **dadurch gekennzeichnet, dass** die Halterung (3) mehrere Trägerelemente (11) aufweist, und dass jedem Trägerelement (11) mindestens eine Strahlungsquelle (7) zugeordnet ist.

7. Strahlereinrichtung nach einem der vorhergehenden Ansprüche, **dadurch gekennzeichnet, dass** diese Teil eines Gesichts-Bräunungsstrahlers ist.

8. Strahlereinrichtung nach einem der vorhergehenden Ansprüche 1 bis 5, **dadurch gekennzeichnet, dass** die Strahlereinrichtung (1) Teil eines Solariums ist.

9. Strahlereinrichtung nach einem der vorhergehenden Ansprüche 1 bis 5, **dadurch gekennzeichnet, dass** die Strahlereinrichtung (1) Teil einer Sonnenwiese ist.

10. Strahlereinrichtung nach einem der vorhergehenden Ansprüche 1 bis 5, **dadurch gekennzeichnet, dass** die Strahlereinrichtung (1) Teil eines Fitness-Centers ist.

## Claims

1. A radiator device of a tanning and/or heat radiating apparatus comprising a fixture (3) and comprising at least one radiation source (7) assigned to the fixture (3) and comprising a drive unit (5), which causes a relative movement between the at least one radiation source (7) and the fixture (3), **characterized in that** the drive unit (5) can be designed as a swivel drive, a circular drive, a rotary drive or a swash drive and comprises an eccentric (19), which engages with the radiation source (7) via at least one actuator (29).

2. The radiator device according to claim 1, **characterized in that** the drive (5) comprises a motor (15).

3. The radiator device according to claim 2, **characterized in that** the eccentric (19) can be realized by means of a specially embodied drive shaft (17) of the motor (25).

4. The radiator device according to claim 3, **characterized in that** the eccentric (19) drives a drive plate (25).

5. The radiator device according to any one of the preceding claims, **characterized in that** the fixture (3) encompasses at least one support element (11) and an elastic connecting element (13), which is affixed to the support element (11) and to the at least one radiation source (7).

6. The radiator device according to any one of the preceding claims, **characterized in that** the fixture (3) encompasses a plurality of support elements (11) and **in that** at least one radiation source (7) is assigned to each support element (11).

7. The radiator device according to any one of the preceding claims, **characterized in that** it is part of a facial tanning radiator.

8. The radiator device according to any one of the preceding claims 1 to 5, **characterized in that** the radiator device (1) is part of a solarium.

9. The radiator device according to any one of the preceding claims 1 to 5, **characterized in that** the radiator device (1) is part of a sun bed.

10. The radiator device according to any one of the preceding claims 1 to 5, **characterized in that** the radiator device (1) is part of a fitness center.

## Revendications

1. Dispositif de rayonnement d'une lampe de bronzage et/ou d'un radiateur thermique avec une fixation (3) et avec au moins une source de rayonnement (7) associée à la fixation (3), et avec un entraînement (5) qui effectue un mouvement relatif entre l'au moins une source de rayonnement (7) et la fixation (3), **caractérisé en ce que** l'entraînement (5) peut être conçu en tant qu'entraînement pivotant, circulaire, centrifuge ou chancelant et comprend un excentrique (19) qui s'applique au moins par le biais d'un poussoir (29) sur la source de rayonnement (7).

2. Dispositif de rayonnement selon la revendication 1, **caractérisé en ce que** l'entraînement (5) comprend un moteur (15).

3. Dispositif de rayonnement selon la revendication 2, **caractérisé en ce que** l'excentrique (19) peut être réalisé par un arbre d'entraînement (17) réalisé spécialement du moteur (25).

4. Dispositif de rayonnement selon la revendication 3, **caractérisé en ce que** l'excentrique (19) entraîne une plaque d'entraînement (25).

5. Dispositif de rayonnement selon l'une quelconque des revendications précédentes, **caractérisé en ce que** la fixation (3) présente au moins un élément de support (11) et un élément de connexion élastique (13) qui est monté sur l'élément de support (11) et sur l'au moins une source de rayonnement (7).

6. Dispositif de rayonnement selon l'une quelconque des revendications précédentes, **caractérisé en ce que** la fixation (3) présente plusieurs éléments de support (11) et qu'au moins une source de rayonnement (7) est associée à chaque élément de support (11).

7. Dispositif de rayonnement selon l'une quelconque des revendications précédentes, **caractérisé en ce que** celui-ci fait partie d'une lampe de bronzage pour le visage.

8. Dispositif de rayonnement selon l'une quelconque des revendications 1 à 5, **caractérisé en ce que** le dispositif de rayonnement (1) fait partie d'un solarium.

9. Dispositif de rayonnement selon l'une quelconque des revendications 1 à 5, **caractérisé en ce que** le dispositif de rayonnement (1) fait partie d'un lit de soleil.

10. Dispositif de rayonnement selon l'une quelconque des revendications 1 à 5, **caractérisé en ce que** le dispositif de rayonnement (1) fait partie d'un centre de remise en forme.
